# EUROPÄISCHE PATENTSCHRIFT

(11) **EP 3 761 947 B1**
(45) Veröffentlichungstag und Bekanntmachung des Hinweises auf die Patenterteilung: **10.08.2022**
(21) Anmeldenummer: 19703275.8
(22) Anmeldetag: 31.01.2019
(51) Int. Cl.: A61K 8/37, A61Q 19/00

(54) **LIPIDMISCHUNG AUS STEARYLBEHENAT UND TRISTEARIN**
LIPID MIXTURE OF STEARYL BEHENATE AND TRISTEARIN
MÉLANGE LIPIDIQUE À BASE DE BÉHÉNATE DE STÉARYLE ET DE TRISTÉARINE

(30) Priorität: 06.03.2018 DE 102018203278
(43) Veröffentlichungstag der Anmeldung: 13.01.2021
(73) Patentinhaber: Beiersdorf AG, 20253 Hamburg (DE)
(72) Erfinder: ZEWUHN, Merle, 25335 Elmshorn (DE); FIEDLER, Dorothe, 22335 Hamburg (DE); RATHSACK, Jessica, 21614 Buxtehude (DE)
(74) Vertreter: Beiersdorf AG
(86) Internationale Anmeldenummer: PCT/EP2019/052339
(87) Internationale Veröffentlichungsnummer: WO 2019/170328

(56) Entgegenhaltungen:
- US-A1- 2010 183 536
- DATABASE GNPD [Online] MINTEL; 18. Februar 2013 (2013-02-18), anonymous: "SOS Repair Body Cream", XP055576360, gefunden im www.gnpd.com Database accession no. 1997468

## Beschreibung

Die vorliegende Erfindung betrifft eine kosmetische Lipidmischung bestehend aus Stearylbehenat und Glycerol-Triestern mit einem Stearinsäure-Anteil von 80%, sowie kosmetische Pasten und Lippenpflegeprodukte (insbesondere Lippenbutter), welche diese Lipidmischung enthalten.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe.

Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

Eine besondere Form kosmetischer Zubereitungen stellen die Lippenpflegeprodukte dar. Sie dienen einerseits der Pflege der Lippenhaut und schützen bzw. regenerieren die Lippen bei Trockenheit und Rissigkeit. Andererseits dienen Lippenpflegeprodukte auch zur Dekoration, da sie den Lippen durch Glanzeffekte oder Farbe zu einem besonderen Ausdruck verhelfen. Lippenpflegeprodukte werden sowohl in Stiftform oder als mehr oder weniger zähfließende Crememasse ("Lippenbutter") dargereicht.

An Lippenpflegeprodukte werden besondere Anforderungen gestellt. Sie müssen in toxikologischer Hinsicht vollkommen unbedenklich sein um jegliche Gefahr einer Vergiftung auszuschließen. Darüber hinaus müssen sie einerseits streichfähig sein, andererseits, nach dem Auftragen auf die Lippen aber dort ortsfest verbleiben ohne klebrig zu wirken. Nicht zuletzt müssen diese Produkte mikrobiologisch und thermisch über einen langen Zeitraum stabil sein. Einen wesentlichen Bestandteil von Lippenpflegeprodukten und anderen Kosmetika stellen seit alters her die Mineralöle und Mineralwachse dar. Diese haben eine angenehme, weiche, cremige Konsistenz, sind geruchslos und geschmacksneutral und sind kostengünstig in großen Mengen von hoher Qualität verfügbar. US 2010/183536 A1 (ANSMANN ACHIM [DE] ET AL) 22. Juli 2010 (2010-07-22) offenbart eine kosmetische Zusammensetzung, enthaltend eine Lipidmischung aus Kohlenwasserstoffe. Es ist möglich, weitere Lipide einzufügen: Glyceryltristearat und Stearylbehenat werden erwähnt.

Ein Nachteil des Standes der Technik besteht nun in dem Umstand, dass der Einsatz von Mineralölen und -wachsen, nicht ganz unumstritten ist und bei Bewertungen bei einigen Verbrauchermagazinen (z.B. Öko-Test) zu "Abwertungen" in der Benotung des Produktes führen. Begründet wird diese Negativ-Bewertung damit, dass einige Wissenschaftler vermuten, dass Mineralöle und Mineralwachse gesundheitlich nicht unbedenklich sein könnten. Auch wenn, trotz des jahrzehntelangen weltweiten Einsatzes dieser Stoffe keine negativen Wirkungen am Menschen bekannt geworden sind, besteht bei den Verbrauchern der Wunsch, Zubereitungen mit derartigen Inhaltsstoffen zu meiden.

Es war daher die Aufgabe der vorliegenden Erfindung, die Nachteile des Standes der Technik zu beseitigen eine alternative Lipidgrundlage (Ersatzstoff) für Mineralöle und, insbesondere, Mineralwachse zu entwickeln, welche die gleichen sensorischen und formulierungstechnischen Eigenschaften wie diese haben.

Wachse, insbesondere Mineralwachse, dienen in kosmetischen Zubereitungen regelmäßig als Ölbinder, welche die flüssigen Ölkomponenten in einer pastösen oder festen Zubereitung fixieren. Allerdings lassen sich nach dem Stand der Technik nicht alle Öle und Wachse gut miteinander verbinden. Häufig kommt es bei thermischer Belastung oder während einer längeren Lagerungsdauer zu Phasentrennungen von Öl und Wachs. Ferner wird immer wieder beobachtet, dass die Wachse teilweise auskristallisieren. Die Zubereitungen oft erscheinen trübe und inhomogen.

Es war daher die Aufgabe der vorliegenden Erfindung, eine Lipidmischung zu entwickeln, in die sich weitere ÖI- und Wachskomponenten problemlos temperatur- und lagerstabil einarbeiten lassen. Insbesondere war es die Aufgabe der vorliegenden Erfindung, eine solche Lipidmischung zu entwickeln, die für Lippenbutter geeignet ist und im Vorratsbehältnis (Tiegel) ein durchgehend homogenes Erscheinungsbild zeigt.

Neben dem Erscheinungsbild der Zubereitung ist es für eine Lippenbutter von Bedeutung, dass sich das Produkt leicht aus dem Tiegel entnehmen lässt. Bei den Produkten des Standes der Technik tritt hingegen immer wieder das Problem auf, dass die Oberfläche der Lippenbutter durch "Austrocknung" fester oder durch "Ausölen" dünnflüssiger wird als die darunter liegenden Produktteile. Es bildet sich eine Art "Haut" auf der Lippenbutter oder ein Ölfilm. Es war daher die Aufgabe der vorliegenden Erfindung, diese Phänomene zu unterdrücken und eine Lippenbutter zu entwickeln, die eine durchgehend homogene, weiche Textur aufweist.

Überraschend gelöst werden die Aufgaben durch eine kosmetische Lipidmischung bestehend aus
a) Stearylbehenat (INCI Stearyl Behenate) und
b) Glycerol-Triester mit einem Stearinsäure-Anteil von 80%.

Dabei gibt die Prozentangabe den Anteil an der eingesetzten Fettsäure (FS)-Menge aus dem Grundrohstoff Öl an.

Dabei ist es erfindungsgemäß bevorzugt, wenn als Glycerol-Triester Tristearin eingesetzt wird. Beispielsweise kann hierfür der Rohstoff Dysan 118-Tristearin der Firma Oleochemicals eingesetzt werden.

Die erfindungsgemäß vorteilhaften Ausführungsformen der vorliegenden Erfindung sind dabei dadurch gekennzeichnet, dass das Gewichts-Verhältnis von Stearylbehenat zu Glycerol-Triester von 1:1,5 bis 1:2,5 beträgt. Erfindungsgemäß besonders bevorzugt ist dabei ein Verhältnis von etwa 1:2.

Erfindungsgemäß ist auch eine kosmetische Paste enthaltend eine erfindungsgemäße Lipidmischung sowie ein oder mehrere kosmetische Öle.

Bei einer solchen kosmetischen Paste ist es erfindungsgemäß vorteilhaft, wenn der Gewichtsanteil an der kosmetischen Lipidmischung von 10 bis 40 Gewichts-% und der Gesamtanteil an einem oder mehreren kosmetischen Ölen von 60 bis 90 Gewichts-%, bezogen auf das Gesamtgewicht der Paste beträgt. Die erfindungsgemäß bevorzugten Einsatzkonzentrationen betragen dabei für die kosmetische Lipidmischung von 15 bis 30 Gewichts-%, bezogen auf das Gesamtgewicht der Paste und für die kosmetischen Öle von 85 bis 70 Gewichts-%, bezogen auf das Gesamtgewicht der Paste.

Dabei ist es erfindungsgemäß von Vorteil, wenn die kosmetischen Öle gewählt werden aus der Gruppe der Verbindungen Octyldodecanol, Sonnenblumenöl, Rizinusöl, Olivenöl, Mandelöl, Ethylhexylstearat, Myristyllactat, Kokosglyceride (INCI: Cocoglycerides), Capryl/Caprinsäure Triglyceride (INCI: Caprylic/ Capric Triglycerides), Triisostearin, Jojobaöl, Avocadoöl.

Erfindungsgemäß bevorzugt sind dabei die kosmetischen Öle Octyldodecanol, Sonnenblumenöl, Rizinusöl, Kokosglyceride, Triisostearin.

Eine erfindungsgemäß besonders bevorzugte erfindungsgemäße Paste hat dabei die folgende Zusammensetzung:
5 bis 10 Gewichts-% Stearylbehenat (INCI Stearyl Behenate)
10 bis 20 Gewichts-% Tristearin
70 bis 85 Gewichts-% eines oder mehrerer kosmetischer Öle gewählt aus der Gruppe der Verbindungen Octyldodecanol, Rizinusöl, Sonnenblumenöl, Kokosglyceride, Triisostearin, wobei sich die Gewichtsangaben auf das Gesamtgewicht der kosmetischen Paste beziehen und sich die Gewichtsangabe der kosmetischen Öle auf die Gesamtmenge dieser Öle.

Erfindungsgemäß bevorzugt ist dabei eine Mischung aus mehreren kosmetischen Ölen. Erfindungsgemäß besonders bevorzugt sind dabei Ölmischungen der folgenden Zusammensetzung:
von 40 bis 60 Gewichts-% Octyldodecanol,
von 15 bis 25 Gewichts-% Rizinusöl,
von 05 bis 20 Gewichts-% Sonnenblumenöl, Kokosglycerides, und/oder Triisostearin,
wobei sich die Gewichtsangaben auf den Gesamtanteil dieser kosmetischen Öle bezieht.

Die erfindungsgemäße Paste stellt also eine Kombination aus erfindungsgemäßer Lipidmischung und kosmetischen Ölen dar.

Erfindungsgemäß ist auch eine kosmetische Lippenbutter enthaltend
a) eine erfindungsgemäße kosmetische Lipidmischung,
b) ein oder mehrere kosmetische Öle,
c) Lipide gewählt aus der Gruppe der Verbindungen Sheabutter, Kakaobutter und/oder Mangobutter.

Diese erfindungsgemäße Lippenbutter enthält also erfindungsgemäß vorteilhaft die erfindungsgemäße Paste und Lipide gewählt aus der Gruppe der Verbindungen Sheabutter, Kakaobutter und/oder Mangobutter. Die erfindungsgemäße kosmetische Paste ist also ein wesentlicher Bestandteil der Lippenbutter.

Dabei ist es erfindungsgemäß besonders bevorzugt, wenn als Lipid Sheabutter eingesetzt wird.

Es ist erfindungsgemäß bevorzugt, wenn die erfindungsgemäße kosmetische Lippenbutter dadurch gekennzeichnet ist, dass der Gewichtsanteil der
Lipidmischung a) von 15 bis 20 Gewichts-% beträgt,
der kosmetischen Öle b) von 25 bis 45 Gewichts-% beträgt und
der Lipide c) von 30 bis 50 Gewichts-% beträgt.

Dabei ist es erfindungsgemäß besonders bevorzugt, wenn die erfindungsgemäße Lippenbutter die folgende Zusammensetzung enthält:
Von 3 bis 8 Gewichts-% Stearylbehenat (INCI Stearyl Behenate)
Von 8 bis 16 Gewichts-% Tristearin
von 35 bis 60 Gewichts-% eines oder mehrerer kosmetischer Öle gewählt aus der Gruppe der Verbindungen Octyldodecanol, Rizinusöl, Triisostearin, Myristyl Lactate.
von 30 bis 50 Gewichts-% Lipide gewählt aus der Gruppe der Verbindungen Sheabutter, Kakaobutter und/oder Mangobutter, wobei sich die Gewichtsangaben auf das Gesamtgewicht der kosmetischen Lippenbutter beziehen und die Gewichtsangaben der kosmetischen Öle und Lipide die jeweilige Gesamtmenge dieser Stoffe angeben. Das bedeutet, dass die Angabe bei lediglich einem Öl bzw. Lipid sich auf dieses Öl bzw. Lipid bezieht und beim Einsatz von Öl- bzw. Lipidmischungen, dies die die Summe aller kosmetischen Öle bzw. Lipide darstellt.

Als erfindungsgemäße kosmetische Öle werden in der Lippenbutter die gleichen Öle in den gleichen Konzentrationen eingesetzt, wie es unter der kosmetischen Paste angegeben ist.

Die erfindungsgemäße Lippenbutter kann als weitere Bestandteile Cetylpalmitat und/oder Cetearylalkohol enthalten. Diese Inhaltsstoffe werden dann in einer Konzentration von 0,5 bis 2,5 Gewichts-%, bezogen auf das Gesamtgewicht der Lippenbutter eingesetzt.

Die erfindungsgemäße kosmetische Paste und die erfindungsgemäße Lippenbutter sind vorteilhafterweise dadurch gekennzeichnet, dass die Zubereitung frei ist von Mineralölen, Mineralwachsen, Silikonölen, Silikonwachsen, Parabenen und Polyethylenglycolen.

Vorteilhafte Ausführungsformen der erfindungsgemäßen kosmetischen Paste und der erfindungsgemäßen Lippenbutter sind dadurch gekennzeichnet, dass die jeweilige Zubereitung einen oder mehrere Farbpigmente enthält.

In einem solchen Falle ist es erfindungsgemäß vorteilhaft, wenn die erfindungsgemäße kosmetische Paste und die erfindungsgemäße Lippenbutter als Zubereitung Farbpigmente in einer Menge von 0,1 bis 1,0 Gewichts-%, bezogen auf das Gesamtgewicht der Zubereitung beträgt.

Diese Farbpigmente können erfindungsgemäß vorteilhaft gewählt werden aus der Gruppe der Verbindungen Verbindungen Cl 15850 (D&C Red No. 7), Cl 15985 (FD&C Yellow No. 6, Cl 17200 (D&C Red No. 33), Cl 42090 (FD&C Blue No. 1), Cl 45380 (D&C Red No. 21), Cl 77266 (D&C Black No. 2), Cl 75470 (Carmine), Cl 77007 (Ultramarines), Cl 77163 (Bismuth Oxycholoride), Cl 77491, Cl 77492, Cl 77499 (Iron Oxides), Cl 77891 (Titanium Dioxide), Mica.

Darüber hinaus sind die erfindungsgemäße kosmetische Paste und die erfindungsgemäße Lippenbutter vorteilhafter Weise dadurch gekennzeichnet, dass die jeweilige Zubereitung einen oder mehrere Aromastoffe und/oder Parfümstoffe enthält.

In einem solchen Falle sind die erfindungsgemäß vorteilhaften Ausführungsformen dadurch gekennzeichnet, dass die jeweilige Zubereitung Aromastoffe bzw. Parfümstoffe in einer Gesamtmenge von 0,1 bis 2,0 Gewichts-%, bezogen auf das Gesamtgewicht der Zubereitung, enthält.

Die erfindungsgemäß vorteilhaften Aromastoffe/Parfümstoffe gewählt werden aus der Gruppe der Verbindungen Acetate und Butyrate. Insbesondere Isopentylacetat ist dabei erfindungsgemäß bevorzugt.

Die erfindungsgemäße Zubereitung, d.h. die kosmetische Paste oder die Lippenbutter können auch weitere lipophile Bestandteile enthalten, beispielsweise Candelillawachs, Carnaubawachs, Bienenwachs, Schellackwachs, Beerenwachs, Hydriertes Jojobawachs, Wollwachs, Zuckerrohrwachs, Reiskeimwachs, Sonnenblumenwachs.

Erfindungsgemäß vorteilhafte Ausführungsformen sind auch dadurch gekennzeichnet, dass die die Zubereitung d.h. die kosmetische Paste oder die Lippenbutter Antioxidantien enthält.

Als erfindungsgemäß bevorzugte Antioxidantien werden dabei BHT (Butylhydroxytoluol), Tocopherol und Tocopherolacetat angesehen.

Die erfindungsgemäße Zubereitung, d.h. die kosmetische Paste oder die Lippenbutter kann bis zu 2 Gewichts-%, bezogen auf das Gesamtgewicht der Zubereitung, an Wasser und/oder hydrophilen Bestandteilen wie Glycerin, Panthenol etc. enthalten.

Für bestimmte Einsatzzwecke kann es erfindungsgemäß von Vorteil sein, wenn die Zubereitung UV-Filter,

Enthält die erfindungsgemäße Zubereitung UV-Filter, so ist es erfindungsgemäß vorteilhaft, wenn die UV-Filter gewählt werden aus der Gruppe der Verbindungen Octocrylen, Homosalat, Ethylhexylsalicylat, Ethylhexylmethoxycinnamat und Butyl Methoxydibenzoylmethan.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Lipidmischung

| Rohstoff | Konzentration (%) |
|---|---|
| Stearyl Behenate | 33,3 |
| Tristearin | 66,6 |

### Kosmetische Paste A (zwei Öle)

| Rohstoff | Konzentration (%) |
|---|---|
| Stearyl Behenate | 10 |
| Tristearin | 20 |
| Octyldodecanol | 45 |
| Rizinusöl | 25 |

### Kosmetische Paste B (ein Öl)

| Rohstoff | Konzentration (%) |
|---|---|
| Stearyl Behenate | 05 |
| Tristearin | 10 |
| Rizinusöl | 85 |

### Kosmetische Paste C (ein Öl)

| Rohstoff | Konzentration (%) |
|---|---|
| Stearyl Behenate | 05 |
| Tristearin | 10 |
| Sonnenblumenöl | 85 |

### Lippenbutter

| Rohstoff | Konzentration (%) |
|---|---|
| Stearyl Behenate | 05 |
| Tristearin | 10 |
| Octyldodecanol | 10,5 |
| Rizinusöl | 20 |
| Triisostearin | 05 |
| Myristyl Lactate | 10 |
| Shea Butter (Butyrospermum Parkii Butter) | 38 |
| Aroma | 01 |
| Pigmente | 0,5 |

## Patentansprüche

1. Kosmetische Lipidmischung bestehend aus
a) Stearylbehenat (INCI Stearyl Behenate) und
b) Glycerol-Triester mit einem Stearinsäure-Anteil von 80%.

2. Kosmetische Lipidmischung nach Anspruch 1, **dadurch gekennzeichnet, dass** als Glycerol-Triester Tristearin eingesetzt wird.

3. Kosmetische Lipidmischung nach einem der vorhergehenden Ansprüche, **dadurch gekennzeichnet, dass** das Gewichts-Verhältnis von Stearylbehenat zu Glycerol-Triester von 1:1,5 bis 1:2,5 beträgt.

4. Kosmetische Paste enthaltend eine Lipidmischung nach einem der vorhergehenden Ansprüche, sowie ein oder mehrere kosmetische Öle.

5. Kosmetische Paste nach Anspruch 4, **dadurch gekennzeichnet, dass** der Gewichtsanteil an der kosmetischen Lipidmischung von 10 bis 40 Gewichts-% und der Gesamtanteil an einem oder mehreren kosmetischen Ölen von 60 bis 90 Gewichts-%, bezogen auf das Gesamtgewicht der Paste beträgt.

6. Kosmetische Paste nach einem der Ansprüche 4 bis 5, **dadurch gekennzeichnet, dass** die kosmetischen Öle gewählt werden aus der Gruppe der Verbindungen Octyldodecanol, Sonnenblumenöl, Rizinusöl, Olivenöl, Mandelöl, Ethylhexylstearat, Myristyllactat, Kokosglyceride (INCI: Cocoglycerides), Capryl/Caprinsäure Triglyceride (INCI: Caprylic/ Capric Triglycerides), Triisotearin, Jojobaöl, Avocadoöl.

7. Kosmetische Lippenbutter enthaltend
a) eine kosmetische Lipidmischung nach Anspruch 1 bis 3,
b) ein oder mehrere kosmetische Öle,
c) Lipide gewählt aus der Gruppe der Verbindungen Sheabutter, Kakaobutter und/oder Mangobutter.

8. Kosmetische Lippenbutter nach Anspruch 7, **dadurch gekennzeichnet, dass** der Gewichtsanteil der
Lipidmischung a) von 15 bis 20 Gewichts-% beträgt,
der kosmetischen Öle b) von 25 bis 45 Gewichts-% beträgt und
der Lipide c) von 30 bis 50 Gewichts-% beträgt.

9. Kosmetische Paste nach Anspruch 4 bis 6 oder Lippenbutter nach Anspruch 7 bis 8, **dadurch gekennzeichnet, dass** die Zubereitung frei ist von Mineralölen, Mineralwachsen, Silikonölen, Silikonwachsen, Parabenen und Polyethylenglycolen.

10. Kosmetische Pasten oder Lippenbutter nach einem der Ansprüche 4 bis 9, **dadurch gekennzeichnet, dass** die Zubereitung einen oder mehrere Farbpigmente enthält.

11. Kosmetische Pasten oder Lippenbutter nach einem der Ansprüche 4 bis 10, **dadurch gekennzeichnet, dass** die Zubereitung Farbpigmente in einer Menge von 0,1 bis 1,0 Gewichts-%, bezogen auf das Gesamtgewicht der Zubereitung beträgt.

12. Kosmetische Paste oder Lippenbutter nach einem der Ansprüche 4 bis 11, **dadurch gekennzeichnet, dass** die Farbpigmente gewählt werden aus der Gruppe der Verbindungen Verbindungen Cl 15850 (D&C Red No. 7), Cl 15985 (FD&C Yellow No. 6, Cl 17200 (D&C Red No. 33), Cl 42090 (FD&C Blue No. 1), Cl 45380 (D&C Red No. 21), Cl 77266 (D&C Black No. 2), Cl 75470 (Carmine), Cl 77007 (Ultramarines), Cl 77163 (Bismuth Oxycholoride), Cl 77491, Cl 77492, Cl 77499 (Iron Oxides), Cl 77891 (Titanium Dioxide), Mica.

13. Kosmetische Paste oder Lippenbutter nach einem der Ansprüche 4 bis 12, **dadurch gekennzeichnet, dass** die Zubereitung einen oder mehrere Aromastoffe und/oder Parfümstoffe enthält.

14. Kosmetische Paste oder Lippenbutter nach einem der Ansprüche 4 bis 13, **dadurch gekennzeichnet, dass** die Zubereitung Aromastoffe bzw. Parfümstoffe in einer Gesamtmenge von 0,1 bis 2 Gewichts-%, bezogen auf das Gesamtgewicht der Zubereitung, enthält.

15. Kosmetische Paste oder Lippenbutter nach einem der Ansprüche 4 bis 14, **dadurch gekennzeichnet, dass** die Aromastoffe/Parfümstoffe gewählt werden aus der Gruppe der Verbindungen Acetate und Butyrate.

## Claims

1. Cosmetic lipid mixture consisting of
a) stearyl behenate (INCI Stearyl Behenate) and
b) glycerine triester having a proportion of stearic acid of 80%.

2. Cosmetic lipid mixture according to Claim 1, **characterized in that** tristearin is used as glycerine triester.

3. Cosmetic lipid mixture according to either of the preceding claims, **characterized in that** the ratio by weight of stearyl behenate to glycerine triester is from 1:1.5 to 1:2.5.

4. Cosmetic paste comprising a lipid mixture according to any of the preceding claims, and one or more cosmetic oils.

5. Cosmetic paste according to Claim 4, **characterized in that** the proportion by weight of the cosmetic lipid mixture is from 10 to 40% by weight and the total proportion of one or more cosmetic oils is from 60 to 90% by weight, based on the total weight of the paste.

6. Cosmetic paste according to either of Claims 4 to 5, **characterized in that** the cosmetic oils are selected from the group of compounds comprising octyldodecanol, sunflower oil, castor oil, olive oil, almond oil, ethylhexyl stearate, myristyl lactate, cocoglycerides (INCI: Cocoglycerides), caprylic/capric acid triglycerides (INCI: Caprylic/Capric Triglycerides), triisostearin, jojoba oil, avocado oil.

7. Cosmetic lip butter comprising
a) a cosmetic lipid mixture according to Claims 1 to 3,
b) one or more cosmetic oils
c) lipids selected from the group of compounds comprising shea butter, cocoa butter and/or mango butter.

8. Cosmetic lip butter according to Claim 7, **characterized in that** the proportion by weight of the lipid mixture a) is from 15 to 20% by weight,
of the cosmetic oils b) is from 25 to 45% by weight and of the lipids c) is from 30 to 50% by weight.

9. Cosmetic paste according to Claims 4 to 6 or lip butter according to Claims 7 to 8, **characterized in that** the preparation is free of mineral oils, mineral waxes, silicone oils, silicone waxes, parabens and polyethylene glycols.

10. Cosmetic pastes or lip butter according to any of Claims 4 to 9, **characterized in that** the preparation comprises one or more colour pigments.

11. Cosmetic pastes or lip butter according to any of Claims 4 to 10, **characterized in that** the preparation comprises colour pigments in an amount of 0.1 to 1.0% by weight, based on the total weight of the preparation.

12. Cosmetic paste or lip butter according to any of Claims 4 to 11, **characterized in that** the colour pigments are selected from the group of compounds comprising Cl 15850 (D&C Red No. 7), Cl 15985 (FD&C Yellow No. 6), Cl 17200 (D&C Red No. 33), Cl 42090 (FD&C Blue No. 1), Cl 45380 (D&C Red No. 21), Cl 77266 (D&C Black No. 2), Cl 75470 (carmine), Cl 77007 (ultramarines), Cl 77163 (bismuth oxychloride), Cl 77491, Cl 77492, Cl 77499 (iron oxides), Cl 77891 (titanium dioxide), mica.

13. Cosmetic paste or lip butter according to any of Claims 4 to 12, **characterized in that** the preparation comprises one or more aroma substances and/or perfumes.

14. Cosmetic paste or lip butter according to any of Claims 4 to 13, **characterized in that** the preparation comprises aroma substances or perfumes in a total amount of 0.1 to 2% by weight, based on the total weight of the preparation.

15. Cosmetic paste or lip butter according to any of Claims 4 to 14, **characterized in that** the aroma substances/perfumes are selected from the group of compounds comprising acetates and butyrates.

## Revendications

1. Mélange lipidique cosmétique consistant en
a) du béhénate de stéaryle (INCl Stearyl Behenate) et
b) un triester du glycérol ayant une proportion d'acide stéarique de 80 %.

2. Mélange lipidique cosmétique selon la revendication 1, **caractérisé en ce qu'**on utilise en tant que triester du glycérol la tristéarine.

3. Mélange lipidique cosmétique selon l'une des revendications précédentes, **caractérisé en ce que** le rapport en poids du béhénate de stéaryle au triester du glycérol est de 1:1,5 à 1:2,5.

4. Pâte cosmétique contenant un mélange lipidique selon l'une des revendications précédentes, ainsi qu'une ou plusieurs huiles cosmétiques.

5. Pâte cosmétique selon la revendication 4, **caractérisée en ce que** la proportion en poids du mélange lipidique cosmétique est de 10 à 40 % en poids et la proportion totale d'une ou plusieurs huiles cosmétiques est de 60 à 90 % en poids, par rapport au poids total de la pâte.

6. Pâte cosmétique selon l'une des revendications 4 à 5, **caractérisée en ce que** les huiles cosmétiques sont choisies dans le groupe des composés octyldodécanol, l'huile de tournesol, huile de ricin, huile d'olive, huile d'amande, stéarate d'éthylhexyle, lactate de myristyle, coco-glycérides (INCI : Cocoglycerides), triglycéride de l'acide caprylique/caprique (INCI : Caprylic/ Capric Triglycerides), triisostéarine, huile de jojoba, huile d'avocat.

7. Beurre à lèvres cosmétique contenant
a) un mélange lipidique cosmétique selon les revendications 1 à 3,
b) une ou plusieurs huiles cosmétiques,
c) des lipides choisis dans le groupe des composés beurre de karité, beurre de cacao et/ou beurre de mangue.

8. Beurre à lèvres cosmétique selon la revendication 7, **caractérisé en ce que** la proportion en poids
du mélange lipidique a) est de 15 à 20 % en poids,
des huiles cosmétiques b) est de 25 à 45 % en poids et
des lipides c) de 30 à 50 % en poids.

9. Pâte cosmétique selon les revendications 4 à 6 ou beurre à lèvres cosmétique selon les revendications 7 à 8, **caractérisée en ce que** la préparation est exempte d'huiles minérales, de cires minérales, d'huiles de silicone, de cires de silicone, de parabens et de polyéthylèneglycols.

10. Pâtes cosmétiques ou beurre à lèvres cosmétique selon l'une des revendications 4 à 9, **caractérisées en ce que** la préparation contient un ou plusieurs pigments colorés.

11. Pâtes cosmétiques ou beurre à lèvres cosmétique selon l'une des revendications 4 à 10, **caractérisées en ce que** la préparation contient des pigments colorés en une quantité de 0,1 à 1,0 % en poids par rapport au poids total de la préparation.

12. Pâte cosmétique ou beurre à lèvres cosmétique selon l'une des revendications 4 à 11, **caractérisée en ce que** les pigments colorés sont choisis dans le groupe des composés Cl 15850 (D&C rouge n° 7), Cl 15985 (FD&C jaune n° 6, Cl 17200 (D&C rouge n° 33), Cl 42090 (FD&C bleu n° 1), Cl 45380 (D&C rouge n° 21), Cl 77266 (D&C noir n° 2), Cl 75470 (carmin), Cl 77007 (bleus outremer), Cl 77163 (oxychlorure de bismuth), Cl 77491, Cl 77492, Cl 77499 (oxydes de fer), Cl 77891 (dioxyde de titane), mica.

13. Pâte cosmétique ou beurre à lèvres cosmétique selon l'une des revendications 4 à 12, **caractérisée en ce que** la préparation contient un ou plusieurs arômes et/ou parfums.

14. Pâte cosmétique ou beurre à lèvres cosmétique selon l'une des revendications 4 à 13, **caractérisée en ce que** la préparation contient des arômes ou des parfums en une quantité totale de 0,1 à 2 % en poids par rapport au poids total de la préparation.

15. Pâte cosmétique ou beurre à lèvres cosmétique selon l'une des revendications 4 à 14, **caractérisée en ce que** les arômes/parfums sont choisis dans le groupe des composés acétates et butyrates.
